(19) Europäisches Patentamt
European Patent Office
Office européen des brevets

(11) **EP 3 263 148 B1**

(12) **EUROPÄISCHE PATENTSCHRIFT**

(45) Veröffentlichungstag und Bekanntmachung des Hinweises auf die Patenterteilung:
**08.05.2019   Patentblatt 2019/19**

(51) Int Cl.:
***A61M 1/10*** *(2006.01)*

(21) Anmeldenummer: **16176858.5**

(22) Anmeldetag: **29.06.2016**

(54) **VERFAHREN ZUR ERMITTLUNG VON BETRIEBSPARAMETERN EINER BLUTPUMPE**

METHOD FOR DETERMINING THE OPERATING PARAMETERS OF A BLOOD PUMP

PROCÉDÉ DE DÉTERMINATION DE PARAMÈTRES DE FONCTIONNEMENT D'UNE POMPE À SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**03.01.2018   Patentblatt 2018/01**

(73) Patentinhaber: **Berlin Heart GmbH 12247 Berlin (DE)**

(72) Erfinder:
• **Granegger, Marcus 10781 Berlin (DE)**
• **Steingräber, Robert 14055 Berlin (DE)**
• **Krone, Jonas Fabian 12167 Berlin (DE)**

(74) Vertreter: **Pfenning, Meinig & Partner mbB Patent- und Rechtsanwälte Joachimsthaler Straße 10-12 10719 Berlin (DE)**

(56) Entgegenhaltungen:
EP-A1- 0 971 212          US-A1- 2005 267 322
US-A1- 2013 030 240      US-A1- 2016 144 092

**Beschreibung**

Die Erfindung liegt auf dem Gebiet der Elektrotechnik und ist mit besonderem Vorteil in der Medizintechnik anwendbar.

Es sind in den vergangenen Jahren mit zunehmendem Erfolg Blutpumpen, insbesondere Herzpumpen, entwickelt worden, die bei Patienten mit vorübergehenden oder dauerhaften Herzproblemen zur Herzunterstützung oder im Extremfall als Ersatz für ein nicht mehr funktionierendes oder schlecht funktionierendes Herz eingesetzt werden können. Solche Pumpen können unterschiedliche Funktionsprinzipien haben. Besonders verbreitet ist der Typ der Rotorpumpe, bei der innerhalb eines Förderkanals ein mittels eines Antriebs rotierend angetriebener Rotor in Axial- oder Radialrichtung Blut fördert.

Bei derartigen Blutpumpen stellt sich die Aufgabe, mit möglichst geringen Mitteln genaue Daten über den erzeugten Blutfluss zu erhalten. Typische Größen, die interessant sind, sind dabei der Blutfluss, d. h. der Volumenstrom durch die Pumpe, die Druckdifferenz über der Pumpe und die Blutviskosität. Üblicherweise sind gerade diese Größen am einfachsten mittels separater Sensoren zu messen, die zwar zur Verfügung stehen, jedoch kostenintensiv sind. Dagegen sind Betriebsparameter der Pumpe meistens in relativ einfacher Weise verfügbar.

Demgemäß sind bereits Verfahren aus dem Stand der Technik bekannt, die dazu benutzt werden, aus Betriebsparametern einer derartigen, meist elektrisch angetriebenen Pumpe Informationen über den Blutfluss zu erhalten (siehe z.B. US 20050267322). Beispielsweise geht aus der WO 2013/003370 A2 ein Verfahren hervor, bei dem die Änderungsrate der Rotordrehzahl erfasst und daraus Informationen über den Blutfluss bezogen werden. Insbesondere können auf diese Weise Informationen über die pulsierende Restherzleistung des unterstützten Herzens gewonnen werden.

Aus der WO 2011/063994 A1 ist ein Verfahren bekannt, bei dem ein Rotor einer Pumpe zu Oszillationsbewegungen angeregt wird, um aufgrund der Analyse mittels eines Modells des Resonators Informationen über die Blutviskosität zu gewinnen.

Die meisten bekannten Verfahren basieren jedoch auf statisch durch Kennlinien beschriebenen Zuständen, d. h. beispielsweise Zusammenhängen von Pumpendrehzahl, Antriebsleistung der Pumpe und der auf einen in Axialrichtung Blut fördernden Rotor wirkenden Kraft oder ähnlichen Größen unter der Voraussetzung eines stabilen Zustands. Schwachstellen einer solchen Annäherung sind einerseits, dass damit die Blutviskosität selbst kaum ermittelt werden kann und demgemäß mit gesonderten Verfahren am Patienten gemessen werden muss und dass solche Modelle das Verhalten der Pumpe in Änderungs- oder Übergangszuständen nicht zu beschreiben vermögen. Solche Zustände sind aber insbesondere zur Gewinnung von Informationen über eine Restherzfunktion sehr wertvoll. Die Restherzfunktion ist naturgemäß pulsierend und führt damit zu schnellen Änderungen der physikalischen Last an der Pumpe.

Der vorliegenden Erfindung liegt vor dem Hintergrund des Standes der Technik die Aufgabe zugrunde, ein Verfahren zur Ermittlung von Betriebsparametern einer Blutpumpe zu schaffen, das auch bei laufender Änderung von Betriebsparametern anwendbar ist.

Die Aufgabe wird durch ein Verfahren mit den Merkmalen des Patentanspruchs 1 gelöst. Die Patentansprüche 2 bis 12 stellen weitere Aspekte der Erfindung dar. Die Aufgabe wird weiter durch eine Blutpumpeneinrichtung gemäß Patentanspruch 13 gelöst, wobei die Unteransprüche 14 und 15 Ausgestaltungen einer solchen Blutpumpeneinrichtung definieren.

Die Erfindung bezieht sich demgemäß auf ein Verfahren zur Ermittlung, insbesondere zur Schätzung, von Betriebsparametern einer Blutpumpe mit einem das Blut fördernden Rotor, bei dem das Änderungsverhalten von wenigstens einem ersten und einem zweiten voneinander unabhängigen Betriebsparameter der Pumpe ermittelt wird und bei dem eine Ermittlung des Flusses durch die Pumpe und/oder einer Druckdifferenz über der Pumpe und/oder der Viskosität des Blutes unter Berücksichtigung des ermittelten Änderungsverhaltens der wenigstens zwei Betriebsparameter erfolgt.

Dadurch, dass für wenigstens zwei erfassbare Betriebsparameter auch zeitliche Änderungsraten laufend erfasst oder ermittelt werden, kann für die modellhafte Beschreibung einer Blutpumpe anders als nach dem Stand der Technik ein dynamisches Modell gewählt werden, das miteinander verknüpfte, unabhängige Differenzialgleichungen nutzt, die jeweils dynamische Abhängigkeiten mehrerer Pumpenparameter beschreiben. Damit ist ein solches Modell auf dynamische Zustände, d. h. Übergangs- oder Änderungszustände der Pumpe, anwendbar. Die zeitlichen Änderungsraten der erfassten Betriebsparameter können entweder unmittelbar gemessen oder über die fortlaufende Erfassung der Betriebsparameter selbst durch Differenzbildung mathematisch ermittelt werden. Dazu sind entsprechende Abtastraten zu wählen. Beispiele für in Frage kommende Beschreibungsmodelle werden weiter unten gegeben.

Es kann beispielsweise vorgesehen sein, dass wenigstens die Drehzahl der Pumpe als erster Betriebsparameter gemessen und eine zeitliche Änderungsrate der Drehzahl bestimmt wird.

Es kann zudem auch noch eine zweite zeitliche Ableitung der Drehzahl bestimmt und berücksichtigt werden. Auf die Beschleunigungswerte des Rotors hat beispielsweise außer dem Trägheitsmoment des Rotors auch die Viskosität des Blutes einen Einfluss, so dass ein entsprechendes Modell einen Beitrag zur Bestimmung der Blutviskosität liefern kann.

Es kann zudem vorgesehen sein, dass bei einer Pumpe als zweiter Betriebsparameter eine Größe gemessen wird, die eine in Axialrichtung auf den Rotor wirkende Kraft repräsentiert, insbesondere eine axiale Auslenkung des Rotors in einem magnetischen Axiallager, und dass ein zeitliches Änderungsverhalten dieser Größe ermittelt wird.

Oft sind Pumpenrotoren, die in Axialrichtung oder in Axial-/Radial-Richtung Blut fördern, in Magnetlagern gelagert, so dass bei einem Magnetlager unmittelbar aus der axialen Auslenkung des Rotors auf die auf diesen wirkende Axialkraft geschlossen werden kann. Im Falle eines aktiv geregelten Magnetlagers (z. B. Nullkraftregelung) kann die auf den Rotor wirkende Axialkraft durch die Lagerposition und die Lagerstromstärke bestimmt werden. Beschleunigungswerte eines Rotors in Axialrichtung sind außer von der Masse des Rotors auch von der Viskosität des Blutes und dynamischen Größen der Blutströmung abhängig, so dass auch diese Beschleunigungswerte einen Zusammenhang mit der Blutviskosität herstellen.

Zudem kann vorgesehen sein, dass neben dem ersten und zweiten Betriebsparameter und ihrem Änderungsverhalten eine Antriebsleistung des Rotors, insbesondere mittels einer Antriebsmotorstromstärke und insbesondere zusätzlich die Stromstärke durch die Lagerspule eines insbesondere axialen Magnetlagers, gemessen und bei der Ermittlung berücksichtigt wird. Werden somit die Antriebsleistung des Rotors, insbesondere ermittelt anhand der Antriebsmotorstromstärke, die Drehzahl und eine die in Axialrichtung auf den Rotor wirkende Kraft repräsentierende Größe gemessen und zeitliche Ableitungen der oben erläuterten Art ebenfalls erfasst oder ermittelt, so können miteinander gekoppelte Differenzialgleichungen, die einen Zusammenhang zwischen diesen Größen herstellen, zumindest näherungsweise gelöst werden. Hierzu können beispielsweise numerische Näherungsverfahren verwendet werden.

Vorteilhafter erscheint die Anwendung von Schätzverfahren unter systematischer Berücksichtigung von Messunsicherheiten und Störgrößen. Dies bedeutet, dass für die Anwendung eines solchen Schätzverfahrens (auch Beobachter genannt) Störgrößen bestimmt werden können, um möglichst effizient eine erfolgreiche Schätzung zu erlangen. Solche Störgrößen können beispielsweise durch Probemessungen ermittelt oder durch Parametrisierung festgelegt werden. Bei einer Parametrisierung kann beispielsweise ein Parametersatz gewählt, die Schätzung durchgeführt und mit einer realen Messung der zu schätzenden Größen (Pumpenfluss, Viskosität und Druckdifferenz über der Pumpe, beispielsweise durchgeführt mit vorübergehend eingesetzten, genau messenden Sensoren) ermittelt werden.

Beispielsweise bietet sich an, dass bei der Ermittlung die beiden miteinander gekoppelten Differenzialgleichungen

$$J \frac{d\omega}{dt} = K\,I_m - B\,\omega - f_T(\omega, Q)$$

und

$$m \frac{d^2 x}{d^2 t} + \mu \frac{dx}{dt} = K\,i\,I_b + kx - f_x(\omega, Q, H)$$

berücksichtigt werden. Dabei bezeichnet J das Trägheitsmoment des Rotors, $\omega$ die Winkelgeschwindigkeit des Rotors, $I_m$ die Motorstromstärke, K eine Drehmomentkonstante, B eine Reibungskonstante und $f_T$ das Lastmoment des Rotors, welches abhängig von der Drehzahl und dem Blutfluss Q. ist. m bezeichnet die Masse die Rotors, x die Axialauslenkung des Rotors im Lager, $\mu$ eine Reibungskonstante, Ki die Stromsteifheit, $I_b$ die Lagerstromstärke eines aktiv geregelten Magnetlagers, kx die passive Magnetkraft des Magnetlagers und $f_x$ eine Kraft, die axial auf den Rotor wirkt und von der Drehzahl, dem Pumpenfluss und der Druckdifferenz H über der Pumpe abhängig ist.

Zusätzlich und zur Verbesserung der Schätzung kann auch vorgesehen sein, dass zusätzlich die Differenzialgleichung

$$L \frac{dQ}{dt} = -H + f_Q(\omega, Q)$$

berücksichtigt wird. Diese Differenzialgleichung beschreibt die Abhängigkeit der zeitlichen Änderung des Pumpenflusses dQ/dt von der Drehzahl $\omega$, der Druckdifferenz über der Pumpe H und einer von der Drehzahl $\omega$ und dem Pumpenfluss abhängigen Größe $f_Q$. $f_Q$ ist ein vom Pumpenfluss und/oder der Drehzahl abhängiger Strömungswiderstand der Pumpe.

Wenigstens eine, allerdings in den meisten Fällen mehrere der Konstanten aus den drei beschriebenen Differenzialgleichungen ist abhängig von der Viskosität. Auch die Funktionen $f_T$, $f_x$ und $f_Q$ können von der Viskosität abhängig sein.

Zusätzlich oder anstelle der zuletzt genannten Differenzialgleichung kann auch vorgesehen sein, dass eine Back-EMF (elektromagnetische Rückwirkung) eines bürstenlosen Antriebsmotors des Rotors gemessen und eine Differenzialgleichung berücksichtigt wird, die die Abhängigkeit der Back-EMF von der Rotorauslenkung in Axialrichtung, der Drehzahl und der Antriebsleistung beschreibt.

Die Spannungsfunktion an einem Motorstator kann folgendermaßen beschrieben werden:

$$V = RI + L\frac{dI}{dt} + f(\omega, x)$$

$f(w, x)$ ist das Back-EMF, R und L der Widerstand und die Induktivität der Spule und I der induzierte Strom in dieser Spule.

Als besonders vorteilhaft erweist es sich, wenn der Fluss durch die Pumpe und/oder die Druckdifferenz über der Pumpe und/oder die Viskosität des Blutes als Lösung der Differenzialgleichungen durch ein Schätzverfahren, insbesondere unter Verwendung eines Kalman-Filters, ermittelt wird. Ein sogenannter Kalman-Filter, insbesondere ein Extended- oder Unscented Kalman-Filter, beschreibt ein Verfahren zur Schätzung von Lösungen beispielsweise der beschriebenen dynamischen Modelle unter Berücksichtigung von Störgrößen. Derartige Kalman-Filter stehen als fertige Softwaremodule zur Verfügung und können für entsprechende Anwendungen geeignet parametrisiert werden. Auch Störgrößen können charakterisiert werden, das heißt, entweder werden diese ebenfalls nach bestem Wissen parametrisiert oder durch Probemessungen mit kontrollierbaren Störungen ermittelt. Nach einer derartigen Einrichtung eines Kalman-Filters kann das Modul mit den laufend erfassten Betriebsparametern versorgt werden, indem diese eingegeben oder elektronisch übermittelt werden, und das Filter gibt daraufhin Schätzwerte für die Lösung der Differenzialgleichungen in der Form von Schätzwerten für den Durchfluss der Pumpe und/oder die Druckdifferenz über der Pumpe und/oder die Viskosität des geförderten Blutes aus.

Weitere in diesem Zusammenhang verwendbare Methoden zur Bestimmung der unbekannten Zustände in einer Blutpumpe sind rekurrente neuronale Netze oder Neuro-Fuzzy-Systeme.

Da das geschilderte Verfahren besonders erfolgreich bei der Beschreibung dynamischer Modelle für eine Blutpumpe ist, kann weiterhin vorgesehen sein, dass die elektrische Antriebsleistung des Rotors und/oder eine Lagerposition des Rotors gezielt verändert werden, um eine dynamische Reaktion der Pumpe zu erzielen. Dadurch, dass die Änderungsgrößen infolge der gezielten Veränderungen beim Betrieb sich von null unterscheiden, kann das erfindungsgemäße Verfahren bei einem solchen Vorgang seine Vorteile voll entfalten und erfolgreich die zu schätzenden Betriebsparameter ermitteln.

Es kann auch vorgesehen sein, dass die infolge der Pulsatilität und/oder Restherzaktivität eines Patientenherzens erfolgenden dynamischen, insbesondere zyklischen Änderungen des Blutstroms und/oder der Betriebsparameter der Pumpe ermittelt werden, um ein zeitliches Änderungsverhalten eines ersten und zweiten Betriebsparameters während einer dynamischen Reaktion der Pumpe zu erfassen. In diesem Fall macht sich das erfindungsgemäße Verfahren die ohnehin vorhandenen zyklischen Änderungen im Betrieb bei einem lebenden Patienten mit Restherzfunktion zunutze, um seine Vorteile bei der Beschreibung dynamischer Pumpensituationen zu nutzen. Sind die in der beschriebenen Weise vorhandenen zyklischen Änderungen der Betriebsparameter beispielsweise zu gering, so können diese durch zyklische Änderungen der genannten Betriebsparameter der Pumpe verstärkt werden, um eine für die Modellanwendung günstige Dynamik zu erzeugen. In Abhängigkeit vom Maß der Änderungen kann die elektrische Antriebsleistung des Rotors und/oder eine Lagerposition des Rotors gezielt verändert werden, um ein aussagekräftiges dynamisches Verhalten der Pumpe erfassen zu können.

Die Erfindung bezieht sich auch auf ein Verfahren zur Ermittlung, insbesondere zur Schätzung von Betriebsparametern einer Blutpumpe mit einem das Blut fördernden Rotor, bei dem Betriebsparameter der Pumpe laufend erfasst werden, wobei mindestens die Back-EMF (ElectroMagnetic Force) eines bürstenlosen Antriebsmotors des Rotors, die Rotorauslenkung in Axialrichtung, die Rotordrehzahl und die Antriebsleistung erfasst werden, auf der Basis einer Differentialgleichung die Größen miteinander verknüpft werden und die Druckdifferenz über der Pumpe und/oder der Fluss durch die Pumpe und/oder die Viskosität des Blutes mittels eines Schätzverfahrens unter Verwendung eines Kalman-Filters ermittelt wird.

Dabei kann die erste genannte Differenzialgleichung auch noch mit der Differenzialgleichung

$$J\frac{d\omega}{dt} = K I_m - B\omega - f_T(\omega, Q)$$

verknüpft werden.

Die Erfindung bezieht sich außer auf ein Verfahren der oben genannten und erläuterten Art auch auf eine Blutpumpeneinrichtung mit einer Blutpumpe mit einem das Blut fördernden Rotor, der in einem magnetischen Axiallager gelagert ist, mit einer Einrichtung zur Erfassung der Drehzahl des Rotors als erstem Betriebsparameter und mit einer Einrichtung zur Erfassung einer Rotorposition in Axialrichtung als zweitem Betriebsparameter sowie mit einer Einrichtung zur Ermittlung des zeitlichen Änderungsverhaltens der Drehzahl und der Rotorposition sowie mit einer Ermittlungseinrichtung, die dazu eingerichtet ist, unter Berücksichtigung des ermittelten Änderungsverhaltens der wenigstens zwei Betriebsparameter den Fluss durch die Pumpe und/oder eine Druckdifferenz über der Pumpe und/oder die Viskosität des Blutes zu ermitteln. Als Blutpumpen kommen dabei unter anderem solche in Frage, die das Blut wenigstens teilweise in Axialrichtung fördern.

Die Ermittlungseinrichtung kann beispielsweise eine Datenverarbeitungseinrichtung, beispielsweise einen Mikrocontroller, umfassen, der erfasste Betriebsparameter und ihre Änderungsgrößen entweder einem Kalman-Filter zuführt oder selbst ein Programmmodul enthält, das die Funktion eines Kalman-Filters realisiert.

Hierzu kann zudem an der Blutpumpeneinrichtung eine Einrichtung zur Erfassung der Antriebsleistung des Rotors, insbesondere durch die Erfassung einer Antriebsmotorstromstärke und insbesondere mit einer Einrichtung zur Erfassung der Lagerstromstärke eines axialen Magnetlagers oder einer Einrichtung der axialen Auslenkung eines Rotors in einem passiven Magnetlager, vorgesehen sein.

Im Folgenden wird die Erfindung anhand von Ausführungsbeispielen in Figuren einer Zeichnung gezeigt und nachfolgend erläutert. Dabei zeigt

Fig. 1     in einem Längsschnitt schematisch eine Blutpumpe,

Fig. 2     die Blutpumpe aus Figur 1 in einer Frontansicht sowie

Fig. 3     schematisch eine Einrichtung zur Durchführung des erfindungsgemäßen Verfahrens.

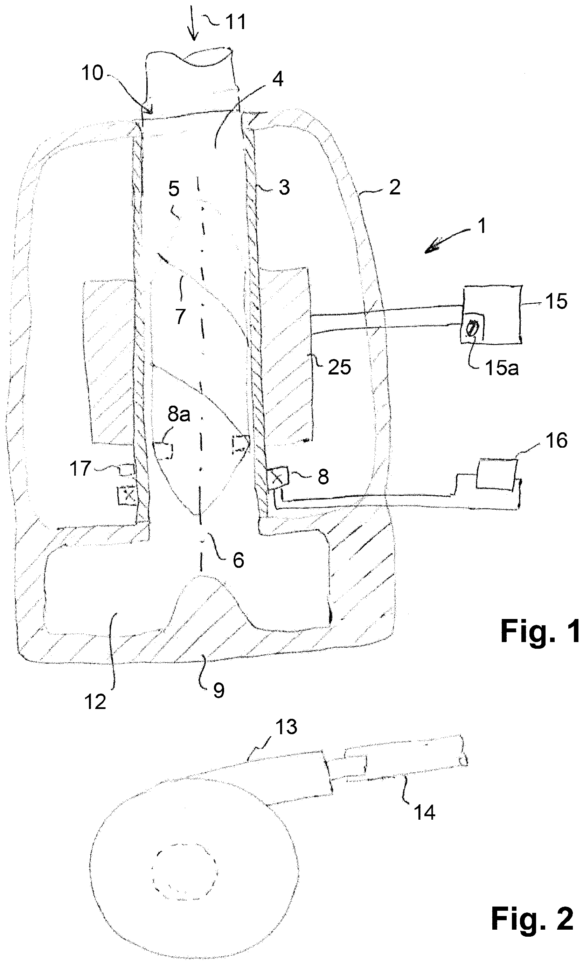

Figur 1 zeigt schematisch eine Blutpumpe 1 mit einem Pumpengehäuse 2, in dem sich ein Pumpenrohr 3 befindet. In dem Pumpenrohr 3 ist rotierend antreibbar ein Rotor 5 gelagert, der ein Förderelement in Form eines wendelförmig um den Rotor 5 umlaufenden Steges 7 aufweist. Der Rotor 5 rotiert, wenn er angetrieben wird, um die Rotationsachse 6. Hierdurch wird am Ende 4 des Rohres, am Pumpeneinlass, durch eine dort beispielsweise angeschlossene Kanüle in Richtung des Pfeils 11 Blut angesaugt. Die Kanüle ist mit 10 bezeichnet und in das Pumpenrohr 3 eingesteckt oder an dieses angedockt.

Im Inneren des Rotors 5 sind (nicht näher dargestellt) Dauermagnete angeordnet, derart, dass der Rotor 5 den Rotor eines bürstenlosen Elektromotors bildet. Der Antrieb des Motors findet mittels geeigneter Ansteuerung von Wicklungen des außenliegenden Stators 25 statt. Dieser kann beispielsweise mit pulswellenmodulierten Signalen angesteuert werden. Die Ansteuereinrichtung ist mit 15 bezeichnet und weist eine Einrichtung 15a zur Erfassung des Antriebsstroms für den Rotor 5 auf.

Der Rotor kann in dem Pumpenrohr radial mittels radialer Magnetlager gelagert sein, die nicht näher dargestellt sind. Es kann auch ein Bereich vorgesehen sein, in dem der Rotor gemeinsam mit dem Pumpenrohr ein hydrodynamisches radiales Lager bildet.

In Axialrichtung ist der Rotor mittels eines magnetischen Axiallagers 8 gelagert, das mit einer Magnetanordnung, insbesondere einem Magnetring 8a, im Rotor wechselwirkt. Das Magnetlager 8 (genauer: dessen stationärer Teil) ist mittels einer Lagerregelung 16 mit einem Lagerstrom beaufschlagt, derart, dass die Position des Rotors 5 in Axialrichtung 6 in die Nullkraftposition geregelt wird. Zu diesem Zweck ist ein Sensor zur Erfassung der Axialposition des Rotors 5 vorgesehen, der mit 17 bezeichnet ist.

Der Rotor 5 fördert das Blut durch das Pumpenrohr 3 in den Auslassraum 12, wo das Blut radial nach außen zu einem Pumpenauslass 13 strömt, der in Figur 2 ersichtlich ist. Vom Pumpenauslass 13 strömt das Blut in eine an diesen angeschlossene Kanüle 14.

In der oben erläuterten Konfiguration ist die Drehzahl des Rotors 5 durch die Ansteuereinrichtung 15 erfassbar. Bei entsprechender Abtastrate kann auch die Änderungsrate, d. h. die erste Ableitung der Drehzahl nach der Zeit, mit ausreichender Genauigkeit bestimmt werden, ebenso wie gegebenenfalls die zweite Ableitung der Drehzahl nach der Zeit. Ebenso kann der Motorstrom durch die Steuereinrichtung in Form der Stromstärke erfasst werden, die beispielsweise über die pulswellenmodulierten Signale integriert werden kann. Die Axialauslenkung des Rotors 5 kann mittels des Hall-Sensors 17 bestimmt werden; ebenso kann bei entsprechender Abtastrate die zeitliche Ableitung $\Delta x/\Delta t$ dieser Größe und die zweite zeitliche Ableitung dieser Größe bestimmt werden. Alternativ oder zusätzlich kann der Lagerstrom durch das geregelte Axiallager 8 als repräsentative Größe für eine auf den Rotor wirkende Axialkraft anstelle der Axialauslenkung in der Regeleinrichtung 16 für den Lagerstrom ermittelt werden.

Damit stehen alle benötigten physikalischen Größen, die variable Betriebsparameter der Pumpe bilden, unmittelbar an der Pumpe zur Verfügung.

Die Parameter Pumpendurchfluss (gefördertes Flüssigkeitsvolumen pro Zeiteinheit), Druckdifferenz über der Pumpe (Differenz der Drücke am Pumpeneinlass 4 einerseits und am Pumpenauslass 13 andererseits) und die Blutviskosität können durch das erfindungsgemäße Verfahren unter Zugrundelegung der erfassten Betriebsparameter durch Schätzung laufend, das heißt auch sehr zeitnah oder ohne zeitlichen Versatz, bestimmt werden.

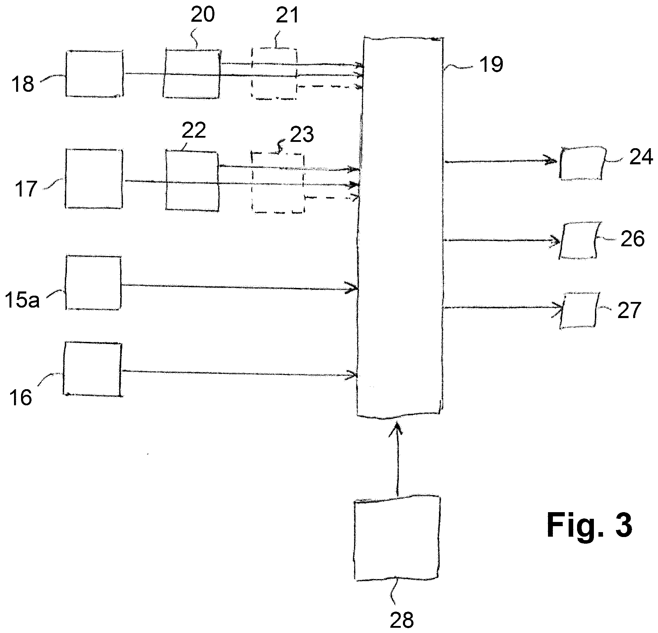

Dies ist schematisch anhand der Figur 3 zu erläutern. In Figur 3 ist der Drehzahlsensor mit 18 bezeichnet. Der Wert der jeweils erfassten Drehzahl wird an die Verarbeitungseinrichtung 19 geleitet. Dabei gelangt der Drehzahlwert an die Einrichtung 20 zur Ermittlung der Änderungsrate der Drehzahl (zeitliche Ableitung). Von dieser wird der augenblickliche Wert der Änderungsrate an die Verarbeitungseinrichtung 19 weitergeleitet. Potenziell erhält auch eine Einrichtung 21 zur Ermittlung der zweiten Ableitung der Drehzahl nach der Zeit den jeweiligen Messwert der Drehzahl und kann die zweite Ableitung ermitteln und, falls diese benötigt wird, an die Verarbeitungseinrichtung 19 weiterleiten.

Der Sensor für die Axialauslenkung des Rotors ist mit 17 bezeichnet und leitet den jeweils momentan erfassten Wert der Auslenkung an die Verarbeitungseinrichtung 19. Der entsprechende Wert wird ebenso an die Verarbeitungseinrichtung 22 geleitet, die die erste zeitliche Ableitung der Axialauslenkung des Rotors ermittelt und diese an die Verarbeitungseinrichtung 19 leitet. Potenziell kann der augenblickliche Wert der Axialauslenkung auch an die Verarbeitungseinrichtung 23 geleitet werden, die die zweite zeitliche Ableitung der Axialauslenkung ermittelt und an die Verarbeitungseinrichtung 19 weiterleitet. Anstelle der Axialauslenkung kann sich diese Konfiguration auch auf eine Lagerstromstärke beziehen.

Der Sensor 15a ermittelt jeweils die Antriebsmotorstromstärke oder einen für diese repräsentativen Wert und leitet diese an die Verarbeitungseinrichtung 19.

Die Regeleinrichtung 16 leitet potenziell den Wert der Lagerstromstärke durch das aktiv geregelte axiale Magnetlager 8 an die Verarbeitungseinrichtung 19.

Die Verarbeitungseinrichtung 19 weist ein Schätzmodul auf, das mithilfe eines Kalman-Filters oder eines Extended-Kalman-Filters laufend Schätzungen der Größen Durchflussrate der Pumpe, Druckdifferenz über der Pumpe und Blutviskosität ermittelt. Die drei Größen werden in den Anzeigen 24, 26 und 27 dargestellt und, falls sinnvoll oder notwendig, gespeichert und/oder an eine weitere Datenverarbeitungseinrichtung weitergeleitet. Es kann auch eine laufende Überwachung der geschätzten Größen auf Über- oder Unterschreitung von Schwellen mit Alarmauslösung vorgesehen sein. Als weitere Eingangsgrößen zusätzlich zu den ermittelten Betriebsparametern verarbeitet die Verarbeitungseinrichtung 19 die durch das Parametrisierungsmodul 28 gelieferten Parameter, die den Einfluss von Störgrößen und Messungenauigkeiten auf den Schätzvorgang repräsentieren. Damit ist unter Verwendung des Kalman-Filters eine zuverlässige Schätzung der genannten Größen möglich.

**Patentansprüche**

1. Verfahren zur Ermittlung, insbesondere zur Schätzung, von Betriebsparametern einer Blutpumpe (1) mit einem das Blut fördernden Rotor (5),

   bei dem das Änderungsverhalten von wenigstens einem ersten und einem zweiten voneinander unabhängigen Betriebsparameter der Pumpe ermittelt wird und bei dem eine Ermittlung des Flusses durch die Pumpe und/oder einer Druckdifferenz über der Pumpe und/oder der Viskosität des Blutes unter Berücksichtigung des ermittelten Änderungsverhaltens der wenigstens zwei Betriebsparameter erfolgt.

2. Verfahren nach Anspruch 1, **dadurch gekennzeichnet, dass** wenigstens die Drehzahl der Pumpe als erster Betriebsparameter gemessen und eine zeitliche Änderungsrate der Drehzahl bestimmt wird.

3. Verfahren nach Anspruch 1 oder 2, **dadurch gekennzeichnet, dass** bei einer Pumpe mit einem das Blut (6) fördernden Rotor (5) als zweiter Betriebsparameter eine Größe gemessen wird, die eine in Axialrichtung (6) auf den Rotor (5) wirkende Kraft (Axialschub) repräsentiert, insbesondere eine axiale Auslenkung des Rotors in einem magnetischen Axiallager (8), und dass ein zeitliches Änderungsverhalten dieser Größe ermittelt wird.

4. Verfahren nach Anspruch 1, 2 oder 3, **dadurch gekennzeichnet, dass** die erste und insbesondere auch die zweite zeitliche Ableitung des zweiten Betriebsparameters ermittelt wird.

5. Verfahren nach Anspruch 1 oder einem der folgenden, **dadurch gekennzeichnet, dass** neben dem ersten und zweiten Betriebsparameter und ihrem Änderungsverhalten eine Antriebsleistung des Rotors (5), insbesondere mittels einer Antriebsmotorstromstärke und insbesondere zusätzlich die Stromstärke durch die Lagerspule eines Magnetlagers (8), gemessen und bei der Ermittlung berücksichtigt wird.

6. Verfahren nach Anspruch 1 oder einem der folgenden, **dadurch gekennzeichnet, dass** bei der Ermittlung die beiden miteinander gekoppelten Differenzialgleichungen

$$J \frac{d\omega}{dt} = K I_m - B\omega - T(\omega, Q)$$

und

$$m \frac{d^2 x}{d^2 t} + \mu \frac{dx}{dt} = K i I_b + kx - f_x(\omega, Q, H)$$

berücksichtigt werden.

7. Verfahren nach Anspruch 6, **dadurch gekennzeichnet, dass** zusätzlich die Differenzialgleichung

$$L \frac{dQ}{dt} = a\omega^2 - H - f_Q(\omega, Q)$$

berücksichtigt wird.

8. Verfahren nach Anspruch 6, **dadurch gekennzeichnet, dass** Back-EMF eines bürstenlosen Antriebsmotors (5, 25) des Rotors (5) gemessen und eine Differenzialgleichung berücksichtigt wird, die die Abhängigkeit der Back-EMF von der Rotorauslenkung in Axialrichtung, der Drehzahl und der Antriebsleistung beschreibt.

9. Verfahren nach Anspruch 6, 7 oder 8, **dadurch gekennzeichnet, dass** der Fluss durch die Pumpe (1) und/oder die Druckdifferenz über der Pumpe und/oder die Viskosität des Blutes als Lösung der Differenzialgleichungen durch ein Schätzverfahren, insbesondere unter Verwendung eines Kalman-Filters, ermittelt wird.

10. Verfahren nach Anspruch 1 oder einem der folgenden, **dadurch gekennzeichnet, dass** die elektrische Antriebsleistung des Rotors (5) und/oder eine Lagerposition des Rotors gezielt verändert werden, um eine dynamische Reaktion der Pumpe zu erzielen.

11. Verfahren nach Anspruch 1 oder einem der folgenden, **dadurch gekennzeichnet, dass** die infolge der Pulsatilität und/oder Restherzaktivität eines Patientenherzens erfolgenden dynamischen Änderungen des Blutstroms und/oder der Betriebsparameter der Pumpe ermittelt werden, um ein zeitliches Änderungsverhalten eines ersten und zweiten Betriebsparameters während einer dynamischen Reaktion der Pumpe zu erfassen.

12. Verfahren zur Ermittlung, insbesondere zur Schätzung von Betriebsparametern einer Blutpumpe mit einem das Blut fördernden Rotor, bei dem Betriebsparameter der Pumpe laufend erfasst werden, wobei mindestens die Back-EMF (ElectroMagnetic Force) eines bürstenlosen Antriebsmotors des Rotors, die Rotorauslenkung in Axialrichtung oder ein für diese repräsentativer Lagerstrom, die Rotordrehzahl und die Antriebsleistung erfasst werden, auf der Basis einer Differentialgleichung die Größen miteinander verknüpft werden und die Druckdifferenz über der Pumpe und/oder der Fluss durch die Pumpe und/oder die Viskosität des Blutes mittels eines Schätzverfahrens unter Verwendung eines Kalman-Filters ermittelt wird.

13. Blutpumpeneinrichtung mit einer Blutpumpe mit einem das Blut wenigstens teilweise in Axialrichtung fördernden Rotor, der in einem magnetischen Axiallager (8) gelagert ist, mit einer Einrichtung zur Erfassung der Drehzahl des Rotors (5) als erstem Betriebsparameter und mit einer Einrichtung zur Erfassung einer Rotorposition in Axialrichtung als zweitem Betriebsparameter sowie mit einer Einrichtung zur Ermittlung des zeitlichen Änderungsverhaltens der Drehzahl und der Rotorposition sowie mit einer Ermittlungseinrichtung (19), die dazu eingerichtet ist, unter Berücksichtigung des ermittelten Änderungsverhaltens der wenigstens zwei Betriebsparameter den Fluss durch die Pumpe und/oder eine Druckdifferenz über der Pumpe und/oder die Viskosität des Blutes zu ermitteln.

14. Blutpumpeneinrichtung nach Anspruch 13, **gekennzeichnet durch** eine Einrichtung zur Erfassung der Antriebsleistung des Rotors, insbesondere durch die Erfassung einer Antriebsmotorstromstärke und insbesondere durch eine Einrichtung zur Erfassung der Lagerstromstärke eines axialen Magnetlagers (8) zusätzlich zu oder anstelle von einer Einrichtung (17) zur Erfassung der axialen Auslenkung eines Rotors in einem passiven magnetischen Axiallager.

**15.** Blutpumpeneinrichtung nach Anspruch 13 oder 14, **gekennzeichnet durch** ein Kalman-Filter.

**Claims**

**1.** A method for determining, in particular for estimating, operational parameters of a blood pump (1) comprising a rotor (5) which transports the blood,
wherein the change in the behaviour of at least one first and one second operational parameter, independently from each other, of the pump, is determined and wherein a determination of the flow through the pump and/or the difference in pressure across the pump and/or the viscosity of the blood takes into account the determined change in behaviour of the at least two operational parameters.

**2.** The method according to claim 1, **characterised in that** at least the rotational speed of the pump is measured as a first operational parameter and a change in rate of the rotational speed over time is determined.

**3.** The method according to claim 1 or 2, **characterised in that** in a pump with a rotor (5) which transports the blood (6) a quantity that represents a force acting on the rotor (5) in the axial direction (6) (axial thrust), in particular an axial deflection of the rotor in a magnetic axial bearing (8), is measured as a second operational parameter, and **in that** a change in behaviour of this quantity over time is determined.

**4.** The method according to claim 1, 2 or 3, **characterised in that** the first and in particular also the second time derivative of the second operational parameter is determined.

**5.** The method according to claim 1 or one of the following claims, **characterised in that** besides the first and second operational parameter and their change in behaviour, a drive power of the rotor (5), in particular by means of a drive motor current strength and in particular additionally the current strength through the bearing coil of a magnetic bearing (8), is measured and is taken into consideration in the determination.

**6.** The method according to claim 1 or one of the following claims, **characterised in that** the two differential equations coupled with one another

$$J\frac{d\omega}{dt} = K\,I_m - B\,\omega - T(\omega, Q)$$

and

$$m\frac{d^2 x}{d^2 t} + \mu\frac{dx}{dt} = K\,i\,I_b + kx - f_x(\omega, Q, H)$$

are taken into consideration in the determination.

**7.** The method according to claim 6, **characterised in that** the differential equation

$$L\frac{dQ}{dt} = a\,\omega^2 - H - f_Q(\omega, Q)$$

is additionally taken into consideration.

**8.** The method according to claim 6, **characterised in that** back EMF of a brushless drive motor (5, 25) of the rotor (5) is measured and a differential equation is taken into consideration which describes the dependency of the back EMF on the rotor deflection in the axial direction, the rotational speed and the drive power.

**9.** The method according to claim 6, 7 or 8, **characterised in that** the flow through the pump (1) and/or the difference in pressure across the pump and/or the viscosity of the blood as solution of the differential equations are/is determined by an estimation method, in particular with use of a Kalman filter.

**10.** The method according to claim 1 or one of the following claims, **characterised in that** the electric drive power of the rotor (5) and/or a bearing position of the rotor are changed selectively in order to attain a dynamic reaction of the pump.

**11.** The method according to claim 1 or one of the following claims, **characterised in that** the dynamic changes in the blood flow and/or the operational parameters of the pump occurring as a result of the pulsatility and/or remaining cardiac activity of a patient's heart are determined in order to detect a change in behaviour over time of a first and second operational parameter during a dynamic reaction of the pump.

**12.** A method for determining, in particular for estimating, operational parameters of a blood pump comprising a rotor which transports the blood, in which method operational parameters of the pump are detected continuously, wherein at least the back EMF (electromagnetic force) of a brushless drive motor of the rotor, the rotor deflection in the axial direction or a bearing current representative of this, the rotor rotational speed and the drive power are detected, the quantities are linked with one another on the basis of a differential equation, and the difference in pressure across the pump and/or the flow through the pump and/or the viscosity of the blood are/is determined by means of an estimation method with use of a Kalman filter.

**13.** A blood pump device comprising a blood pump comprising a rotor which transports the blood at least partially in the axial direction and which is mounted in a magnetic axial bearing (8), comprising a device for detecting the rotational speed of the rotor (5) as a first operational parameter and comprising a device for detecting a rotor position in the axial direction as a second operational parameter, and comprising a device for determining the change in behaviour over time of the rotational speed and the rotor position, and comprising a determination device (19) which is designed to determine the flow through the pump and/or the difference in pressure across the pump and/or the viscosity of the blood under consideration of the determined change in behaviour of the at least two operational parameters.

**14.** The blood pump device according to claim 13, **characterised by** a device for detecting the drive power of the rotor, in particular by the detection of a drive motor current strength, and in particular by a device for detecting the bearing current strength of an axial magnetic bearing (8) in addition to or instead of a device (17) for detecting the axial deflection of a rotor in a passive magnetic axial bearing.

**15.** The blood pump device according to claim 13 or 14, **characterised by** a Kalman filter.

## Revendications

**1.** Procédé de détermination, en particulier d'évaluation, de paramètres de fonctionnement d'une pompe à sang (1) dotée d'un rotor (5) propulsant le sang,
chez lequel le comportement de modification d'au moins un premier et d'un second paramètres de fonctionnement, indépendants l'un de l'autre, est déterminé et chez lequel une détermination de l'écoulement a lieu par la pompe, et/ou une différence de pression au niveau de la pompe et/ou de la viscosité du sang, moyennant la prise en compte du comportement de modification déterminé des au moins deux paramètres de fonctionnement.

**2.** Procédé selon la revendication 1, **caractérisé en ce qu'**au moins le nombre de tours de la pompe est mesuré en tant que premier paramètre de fonctionnement et un taux de modification dans le temps du nombre de tours est déterminé.

**3.** Procédé selon la revendication 1 ou la revendication 2, **caractérisé en ce que**, pour une pompe dotée d'un rotor (5) propulsant le sang (6), une grandeur est mesurée en tant que deuxième paramètre de fonctionnement, qui représente une force (une poussée axiale) agissant sur le rotor (5) dans la direction axiale (6), en particulier une déviation axiale du rotor dans un palier axial (8) magnétique, et que le comportement de modification dans le temps de cette grandeur est mesuré.

**4.** Procédé selon les revendications 1, 2 ou 3, **caractérisé en ce que** la dérivée première dans le temps et, en particulier, la dérivée seconde dans le temps, du deuxième paramètre de fonctionnement sont déterminées.

**5.** Procédé selon la revendication 1 ou l'une des suivantes, **caractérisé en ce qu'**à côté des premier et deuxième paramètres de fonctionnement et de leur comportement de modification, une puissance d'entraînement du rotor (5) est mesurée en particulier au moyen d'une intensité de courant de moteur d'entraînement et en particulier, en plus, par une intensité de courant par la bobine de palier d'un palier magnétique (8) et il en est tenu compte lors de la détermination.

6. Procédé selon la revendication 1 ou l'une des suivantes, **caractérisé en ce que**, lors de la détermination, il est tenu compte des deux équations différentielles couplées conjointes

$$J \frac{d\omega}{dt} = K\, I_m - B\,\omega - T\left(\omega, Q\right)$$

et

$$m \frac{d^2 x}{d^2 t} + \mu \frac{dx}{dt} = K\, i\, I_b + kx - f_x\left(\omega, Q, H\right).$$

7. Procédé selon la revendication 6, **caractérisé en ce qu'**en outre on tient compte de l'équation différentielle

$$L \frac{dQ}{dt} = a\,\omega^2 - H - f_Q\left(\omega, Q\right).$$

8. Procédé selon la revendication 6, **caractérisé en ce qu'**une force contre-électromotrice d'un moteur d'entraînement (5, 25) sans balais du rotor (5) est mesuré et qu'on tient compte d'une équation différentielle qui décrit la dépendance de la force contre-électromotrice avec la déviation de rotor dans la direction axiale, le nombre de tours et la puissance d'entraînement.

9. Procédé selon les revendications 6, 7 ou 8, **caractérisé en ce que** l'écoulement à travers la pompe (1), et/ou la différence de pression au niveau de la pompe, et/ou la viscosité du sang, sont déterminées en tant que solutions des équations différentielles par un procédé d'évaluation, en particulier, moyennant l'utilisation d'un filtre de Kalman.

10. Procédé selon la revendication 1 ou l'une des suivantes, **caractérisé en ce que** la puissance d'entraînement électrique du rotor (5) et/ou une position de palier du rotor sont modifiées de manière ciblée afin d'atteindre une réaction dynamique de la pompe.

11. Procédé selon la revendication 1 ou l'une des suivantes, **caractérisé en ce qu'**en conséquence de la pulsatilité et/ou de l'activité cardiaque résiduelle d'un coeur de patient, des modifications dynamiques du flux sanguin et/ou des paramètres de fonctionnement de la pompe résultantes sont déterminées afin de détecter un comportement de modification dans le temps d'un premier et d'un second paramètre de fonctionnement pendant une réaction dynamique de la pompe.

12. Procédé de détermination, en particulier d'évaluation, de paramètres de fonctionnement d'une pompe à sang dotée d'un rotor propulsant le sang, chez lequel des paramètres de fonctionnement de la pompe sont détectés en continu, où au moins la force contre-électromotrice (force électromagnétique) d'un moteur d'entraînement sans balais du rotor, la déviation du rotor dans la direction axiale ou un courant de palier représentatif de celles-ci, le nombre de tours et la puissance d'entraînement sont détectés, sont reliés entre eux sur la base d'une équation différentielle des grandeurs et la différence de pression au niveau de la pompe, et/ou le flux à travers la pompe, et/ou la viscosité du sang, sont déterminés au moyen d'un procédé d'évaluation, moyennant l'utilisation d'un filtre de Kalman.

13. Dispositif de pompe à sang doté d'une pompe à sang avec un rotor, qui est logé dans un palier axial (8) magnétique, propulsant le sang au moins partiellement en direction axiale, doté d'un dispositif pour la détection du nombre de tours du rotor (5) en tant que premier paramètre de fonctionnement et doté d'un dispositif pour la détection d'une position de rotor en direction axiale en tant que deuxième paramètre de fonctionnement, ainsi que doté d'un dispositif pour la détermination du comportement de modification dans le temps du nombre de tours et de la position du rotor, ainsi que doté d'un dispositif de détermination (19) qui est conçu, en tenant compte du comportement de modification déterminé des au moins deux paramètres de fonctionnement, pour déterminer le flux à travers la pompe, et/ou une différence de pression au niveau de la pompe, et/ou la viscosité du sang.

14. Dispositif de pompe à sang selon la revendication 13, **caractérisé par** un dispositif de détection d'une puissance d'entraînement du rotor, en particulier par la détection d'une intensité de courant de moteur d'entraînement et en particulier, par un dispositif de détection de l'intensité de courant de palier d'un palier magnétique (8) axial, en complément ou à la place d'un dispositif (17) destiné à la détection de la déviation axiale d'un rotor dans un palier axial magnétique passif.

15. Dispositif de pompe à sang selon la revendication 13 ou la revendication 14, **caractérisé par** un filtre de Kalman.

**Fig. 1**

**Fig. 2**

**Fig. 3**

**IN DER BESCHREIBUNG AUFGEFÜHRTE DOKUMENTE**

**In der Beschreibung aufgeführte Patentdokumente**

- US 20050267322 A
- WO 2013003370 A2
- WO 2011063994 A1